# EUROPEAN PATENT APPLICATION

(11) **EP 2 514 481 A1**
(43) Date of publication of application: **24.10.2012**
(21) Application number: 10837369.7
(22) Date of filing: 02.11.2010
(51) Int. Cl.: A61N 1/372, A61N 1/34

(54) **ELECTRIC STIMULATION DEVICE**

(30) Priority: 14.12.2009 JP 2009283338; 24.02.2010 JP 2010039339
(71) Applicant: Terumo Kabushiki Kaisha, Tokyo 151-0072 (JP)
(72) Inventor: FUKUI Yoshihito, Ashigarakami-gun Kanagawa 259-0151 (JP); UNO Takuya, Ashigarakami-gun Kanagawa 259-0151 (JP); ONODA Masahiro, Ashigarakami-gun Kanagawa 259-0151 (JP)
(74) Representative: Dossmann, Gérard
(86) International application number: PCT/JP2010/069482
(87) International publication number: WO 2011/074339

(57) **Abstract**

An external feeding device for feeding to an electric stimulation device can be reduced in size. The electric stimulation device is equipped with a main body block, which has a stimulation circuit block and a support body. The stimulation circuit block has a stimulation electrode for stimulating nerves in a living body or muscles of a living body and a stimulation circuit electrically connected to the stimulation electrode and applying a stimulation signal to the stimulation electrode. The support body is connected to the stimulation circuit block and holds the implant position of the stimulation electrode in the living body. The electric stimulation device is further equipped with a fixation body formed so as to be connectable to the base end side of the main body block. The fixation body includes a fixation-body-side coil portion for receiving electromagnetic waves oscillated from an external device. When the fixation body is connected to the main body block, the fixation-body-side coil portion is electrically connected to the stimulation circuit, which makes it possible to perform feeding depending on the electromagnetic waves to and/or communication with the stimulation circuit.

## Description

### Technical Field

The present invention relates to an electric stimulation device which electrically stimulates a living body, and particularly, to an electric stimulation device which is used while being completely implanted into a living body.

### Background Art

Currently, in a case where there is no effect in conventional medication therapy, nerve block therapy, or surgical therapy in pain treatment or the treatment may not be continuously performed due to adverse effects, it is effective to perform an electric stimulation treatment which relieves pain by electrically stimulating nerves. Electrical spinal cord stimulation treatment which is one of the electric stimulation treatments is a stimulation treatment which electrically stimulates the spinal cord in order to relieve pain transmitted to the brain through the spinal cord.

In the electrical spinal cord stimulation treatment, generally, a trial period is provided from 24 hours to several weeks so as to check the effectiveness of reliving pain through the electric stimulation. In the trial period, generally, a stimulation electrode is placed in an epidural space present outside a spinal dura mater covering the spinal cord through a puncture at the back side, and an electrode lead having the stimulation electrode is connected to a stimulation device at the outside of the living body. In this state, a pain relieving degree is examined under various stimulation patterns. In this period, the implantation of the electric stimulation device is not performed. Only when a predetermined effect is found in the trial period, the implantation of the electric stimulation device is performed.

In a case where the implantation of the electric stimulation device is performed, the electrode lead placed therein during the trial period is extracted, a new stimulation electrode is placed in the epidural space again, and the electrode lead including the stimulation electrode is guided to the lumbar, the abdomen, or the chest by a subcutaneous tunnel. Then, the electrode lead is connected to the electric stimulation device and the electric stimulation device is implanted subcutaneously therein.

Incidentally, in the trial period of the electrical spinal cord stimulation treatment, since the electrode lead is connected to the electric stimulation device at the outside of the living body, there are problems in which a risk of infection occurs, the activity of a patient is limited, or the estimation on the effectiveness of reliving pain is affected by the stress caused by the limitation in activity.

On the other hand, in the technique disclosed in PTL 1, a leadless micro stimulation device having electrodes at both ends of a housing is disclosed. When the micro stimulation device is implanted to the vicinity of a nerve, it is possible to reduce a risk of infection and to reduce the limitation of the patient as much as possible.

### Citation List

### Patent Literature

[PTL 1] US Patent No. 5,193,539

### Summary of Invention

### Technical Problem

The micro stimulation device disclosed in PTL 1 includes a coil therein, and obtains electric power through electromagnetic induction with a coil disposed at the outside of the living body. However, when the micro stimulation device is placed inside a deep lumen, for example, an epidural space positioned generally at the depth of about 5 cm from the skin, there is a need to transmit large electric power from the coil disposed at the outside of the living body so as to obtain the desired electric power, and a power feeding device including the coil at the outside of the living body increases in size.

The invention is made in view of such environments, and it is an object of the invention to provide an electric stimulation device enabling power feeding or communication even in a lumen such as an epidural space and capable of decreasing the size of an external power feeding device used for power feeding.

### Solution to Problem

In order to solve the above-described problem, an electric stimulation device of the invention is formed in a bendable elongated shape and is implanted into a living body. The electric stimulation device includes a main body block that includes a stimulation electrode stimulating nerves or muscles inside the living body, a stimulation circuit block with a stimulation circuit electrically connected to the stimulation electrode and applying a stimulation signal to the stimulation electrode, and a support body connected to the stimulation circuit block and holding the implant position of the stimulation electrode inside the living body. Further, the electric stimulation device includes a fixing body that is formed so as to be connectable to the base end portion of the main body block. The fixing body includes a fixing body side coil portion that receives an electromagnetic wave transmitted from an external device. Then, when the fixing body is connected to the main body block, the fixing body side coil portion is electrically connected to the stimulation circuit, so that power feeding and/or communication with respect to the stimulation circuit in response to the electromagnetic wave are enabled.

According to the above-described configuration of the invention, when the fixing body is connected to the main body block, the fixing body side coil portion may be disposed in the support body side while the fixing body side coil portion and the stimulation circuit are electrically connected to each other.

### Advantageous Effects of Invention

According to the invention, since the fixing body side coil portion is disposed in the support body side, the fixing body side coil portion may be implanted to the vicinity right below the skin when the electric stimulation device is completely implanted into the living body. Accordingly, it is possible to reduce the magnitude of the electromagnetic wave necessary for the fixing body side coil portion to generate electric power through electromagnetic induction. As a result, it is possible to obtain an advantage that the external device (the external power feeding device) transmitting an electromagnetic wave to the fixing body side coil portion may be decreased in size.

### Brief Description of Drawings

[Fig. 1] Fig. 1 is a perspective view illustrating an entire electric stimulation device according to a first embodiment of the invention.
[Fig. 2] Figs. 2A to 2C are exploded external views illustrating an entire main body block which constitutes the electric stimulation device according to the first embodiment of the invention.
[Fig. 3] Fig. 3A is an enlarged diagram illustrating the main body block according to the first embodiment of the invention. Fig. 3B is a cross-sectional view illustrating the main body block according to the first embodiment of the invention in the axial direction.
[Fig. 4] Figs. 4A to 4G are cross-sectional views illustrating the main body block according to the first embodiment of the invention in the radial direction.
[Fig. 5] Fig. 5A is an enlarged diagram illustrating a fixing body according to the first embodiment of the invention. Fig. 5B is an enlarged cross-sectional view illustrating a part of the fixing body according to the first embodiment of the invention.
[Fig. 6] Fig. 6 is a diagram illustrating a state where the fixing body is inserted into a support body of the electric stimulation device according to the first embodiment of the invention.
[Fig. 7] Fig. 7A is a diagram illustrating the length and the arrangement of an electric power feeding electrode and a fixing body electrode according to the first embodiment of the invention. Fig. 7B is a diagram illustrating a connection state of the electric power feeding electrode and the fixing body electrode according to the first embodiment of the invention.
[Fig. 8] Fig. 8 is a block diagram illustrating a functional configuration of a stimulation circuit and a fixing body according to the first embodiment of the invention.
[Fig. 9] Fig. 9 is a diagram illustrating a procedure of implanting the electric stimulation device according to the first embodiment of the invention into a living body.
[Fig. 10] Fig. 10 is a diagram illustrating a procedure of implanting the electric stimulation device according to the first embodiment of the invention into the living body.
[Fig. 11] Fig. 11 is a diagram illustrating a procedure of implanting the electric stimulation device according to the first embodiment of the invention into the living body.
[Fig. 12] Fig. 12 is a diagram illustrating a procedure of implanting the electric stimulation device according to the first embodiment of the invention into the living body.
[Fig. 13] Fig. 13 is a diagram illustrating a procedure of implanting the electric stimulation device according to the first embodiment of the invention into the living body.
[Fig. 14] Fig. 14 is a diagram illustrating a procedure of implanting the electric stimulation device according to the first embodiment of the invention into the living body.
[Fig. 15] Fig. 15 is a block diagram illustrating a functional configuration of a power feeding patch according to the first embodiment of the invention.
[Fig. 16] Fig. 16 is a perspective view illustrating an entire electric stimulation device according to a second embodiment of the invention.
[Fig. 17] Figs. 17A to 17C are exploded external views illustrating an entire main body block which constitutes the electric stimulation device according to the second embodiment of the invention.
[Fig. 18] Fig. 18A is an enlarged diagram illustrating the main body block according to the second embodiment of the invention. Fig. 18B is a cross-sectional view illustrating the main body block according to the second embodiment of the invention in the axial direction.
[Fig. 19] Fig. 19 is a perspective view illustrating an entire electric stimulation device according to a third embodiment of the invention.
[Fig. 20] Figs. 20A to 20D are exploded external views illustrating the entire electric stimulation device according to the third embodiment of the invention.
[Fig. 21] Fig. 21A is an enlarged diagram illustrating a main body block according to the third embodiment of the invention. Fig. 21B is a cross-sectional view illustrating the main body block according to the third embodiment of the invention in the axial direction.
[Fig. 22] Figs. 22A to 22E are cross-sectional views illustrating the main body block according to the third embodiment of the invention in the radial direction.
[Fig. 23] Fig. 23A is an enlarged diagram illustrating a fixing body according to the third embodiment of the invention. Fig. 23B is a cross-sectional view illustrating the fixing body according to the third embodiment of the invention in the axial direction. Fig. 23C is an enlarged diagram of a top surface when the fixing body according to the third embodiment of the invention is seen from the base end portion. Fig. 23D is an enlarged diagram of a bottom surface when the fixing body according to the third embodiment of the invention is seen from a front end portion.
[Fig. 24] Fig. 24 is a block diagram mainly illustrating a stimulation circuit of the electric stimulation device according to the third embodiment of the invention.
[Fig. 25] Fig. 25 is a diagram illustrating a procedure of implanting the electric stimulation device according to the third embodiment of the invention into the living body.
[Fig. 26] Fig. 26 is a diagram illustrating a procedure of implanting the electric stimulation device according to the third embodiment of the invention into the living body.
[Fig. 27] Fig. 27 is a diagram illustrating a procedure of implanting the electric stimulation device according to the third embodiment of the invention into the living body.
[Fig. 28] Fig. 28 is a perspective view illustrating an electric stimulation device according to a fourth embodiment of the invention.
[Fig. 29] Fig. 29 is an enlarged cross-sectional view mainly illustrating a fixing body according to the fourth embodiment of the invention in the axial direction. Description of Embodiments

Hereinafter, examples of embodiments of the invention will be described. The embodiments to be described below are appropriate specific examples of the invention. For this reason, the embodiments have various limitations which are desirable in the technical viewpoint. However, the scope of the invention is not limited to such embodiments unless there is a particular remark for limiting the invention. For example, the numerical conditions of respective parameters exemplified in the following description are merely appropriate examples, and the dimensions, the shapes, and the arrangement relationships in respective drawings used for description are also schematically set.

The invention will be described according to the following procedure.

### <Description of example of first embodiment>

(1) Configuration of electric stimulation device
(2) Configuration of stimulation circuit and the like
(3) Procedure of implanting electric stimulation device
(4) Configuration of power feeding patch

### <Description of example of second embodiment>

(5) Configuration of electric stimulation device
(6) Procedure of implanting electric stimulation device

### <Description of example of third embodiment>

(7) Configuration of electric stimulation device
(8) Configuration of stimulation circuit and the like
(9) Procedure of implanting electric stimulation device

### <Description of example of fourth embodiment>

(10) Configuration of electric stimulation device

### <Description of modified example>

### <Description of first embodiment of the invention>

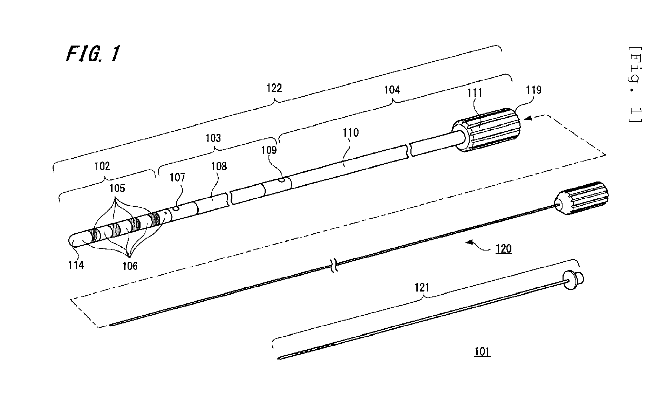

An example of a first embodiment of the invention will be described by referring to Fig. 1 to Fig. 15.

### [1. Configuration of electric stimulation device]

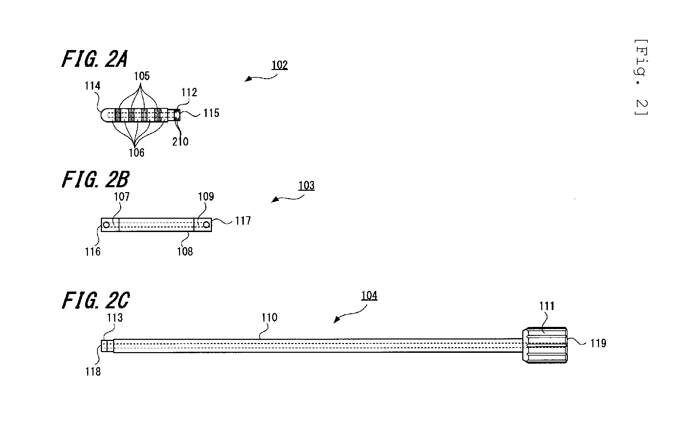

First, a schematic configuration of an electric stimulation device according to the first embodiment will be described by referring to Fig. 1 and Figs. 2A to 2C.

Fig. 1 is a perspective view illustrating the entire electric stimulation device according to the first embodiment.

Figs. 2A to 2C are exploded external views when a main body block constituting the electric stimulation device shown in Fig. 1 is seen from the top surface thereof.

An electric stimulation device 101 is formed in a substantially elongated cylindrical shape and generates an electric stimulation signal so as to stimulate nerves and the like inside a living body by the stimulation signal. The electric stimulation device 101 is implanted into the living body (for example, an epidural space in which the distance between the spinal dura mater and the ligamentum flavum is about 5 mm) when stimulating nerves of the spinal cord. For this reason, it is desirable that the electric stimulation device 101 be formed so that a diameter from a front end portion 114 to a predetermined portion of a support body 104 (to be described later) is about 1 mm to 3 mm.

The electric stimulation device 101 is largely classified into an electrode block 102, a circuit block 103, the support body 104, and a fixing body 121. Then, the electrode block 102 and the circuit block 103 are adapted to be attachable to and detachable from each other at a connector portion 107, and the circuit block 103 and the support body 104 are adapted to be attachable to and detachable from each other at a connector portion 109. In the first embodiment, the portion which is formed by connecting the electrode block 102, the circuit block 103, and the support body 104 to each other is referred to as a main body block 122.

The connection of respective blocks 102, 103, and 104 will be described in more detail.
As shown in Figs. 2A to 2C, with regard to the electrode block 102 and the circuit block 103, a connector portion 112 near the electrode block 102 and the connector portion 107 near the circuit block 103 are fixed by, for example, a screw or the like. In the same way, with regard to the circuit block 103 and the support body 104, the connector portion 109 near the circuit block 103 and a connector portion 113 near the support body 104 are fixed by a screw or the like. In a case where the electrode block 102, the circuit block 103, and the support body 104 are connected to each other, these respective blocks have a substantially cylindrical hole (hereinafter, referred to as a "stylet lumen") through which a stylet 120 passing the blocks in the axial direction is inserted. However, the stylet lumen is opened to a base end portion 119 and is provided up to the vicinity of the front end portion 114. Furthermore, it is desirable that the diameter of the stylet lumen be substantially equal to or slightly larger than the diameter of the stylet 120. Further, the stylet lumen through which the stylet passes may be used as a lumen through which a medical instrument, a drug, or the like passes.

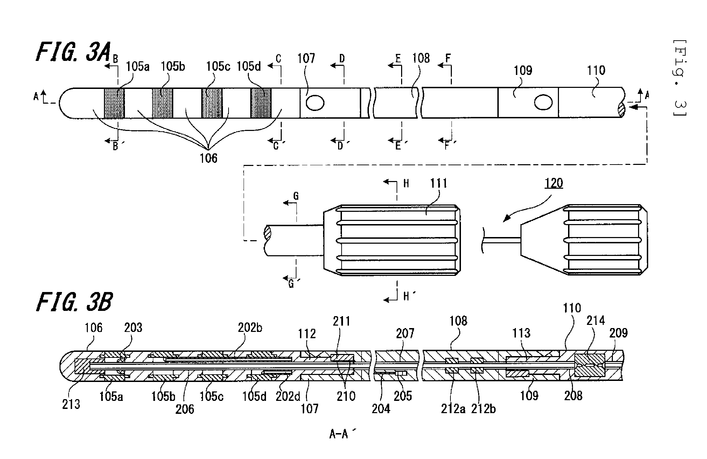

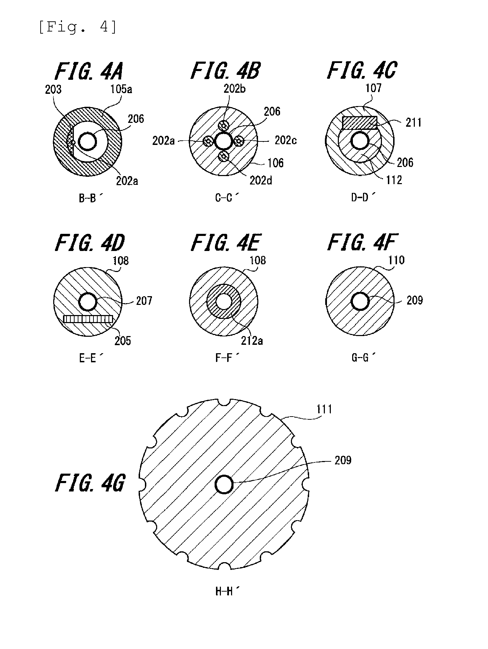

With regard to the electrode block 102, the front end portion 114 is formed in a substantially semi-spherical shape, and the remaining portion is formed in a substantially cylindrical shape. It is desirable that the radius of the substantially semi-spherical portion of the front end portion 114 be about 0.5 mm to 1.5 mm and the diameter of the remaining substantially cylindrical portion be about 1 mm to 3 mm. The electrode block 102 includes four stimulation electrodes 105 which stimulate nerves and the like and bodies 106 which are arranged at the same interval so that the respective stimulation electrodes 105 are exposed to the living body when the electric stimulation device 101 is implanted inside the living body. Furthermore, the electrode block 102 includes the connector portion 112 which connects a base end portion 115 of the body 106 and a front end portion 116 of the circuit block 103 so that they are continuous to each other. Furthermore, in the example of the first embodiment, the number of the stimulation electrodes 105 is exemplified as four, but this is merely an example, and the number of the stimulation electrodes 105 may be arbitrarily set. The internal configuration of the electrode block 102 will be described later in Figs. 3A and 3B and Figs. 4A to 4G.

The circuit block 103 is formed in a substantially cylindrical shape so as to have the same diameter as that of the electrode block 102. The circuit block 103 includes the connector portion 107 which is connected to the connector portion 112 of the electrode block 102 so that the front end portion 116 is continuous to the body 106 near the base end portion 115 of the electrode block 102. Further, the circuit block 103 is equipped with a body 108 which is continuous to the connector portion 107. Furthermore, the circuit block 103 includes the connector portion 109 which is continuous with the base end portion 117 of the body 108 and connects the base end portion 117 and the support body 104. Furthermore, the internal configuration of the circuit block 103 will be described later by referring to Figs. 3A and 3B and Figs. 4A to 4G.

The support body 104 includes the connector portion 113 which is connected to the circuit block 103, a body 110 which is formed in a substantially cylindrical shape so as to have the same diameter as that of the electrode block 102, and a substantially cylindrical holder portion 111 which has a diameter larger than that of the body 110.

The connector portion 113 of the support body 104 is connected to the connector portion 109 of the circuit block 103 so that a front end portion 118 of the body 110 is continuous to the circuit block 103. The body 110 is a portion which connects the connector portion 113 to the holder portion 111 disposed near the base end portion 119. The holder portion 111 corresponds to a position which is used by a doctor to insert the electric stimulation device 101 into the living body. Furthermore, the body 110 is cuttable so that the electric stimulation device 101 is completely implanted into the living body. Then, when the body 110 is cut, the fixing body 121 may be inserted into the stylet lumen which is opened to the cut surface. Furthermore, the internal configuration of the support body 104 will be described later by referring to Figs. 3A and 3B and Figs. 4A to 4G.

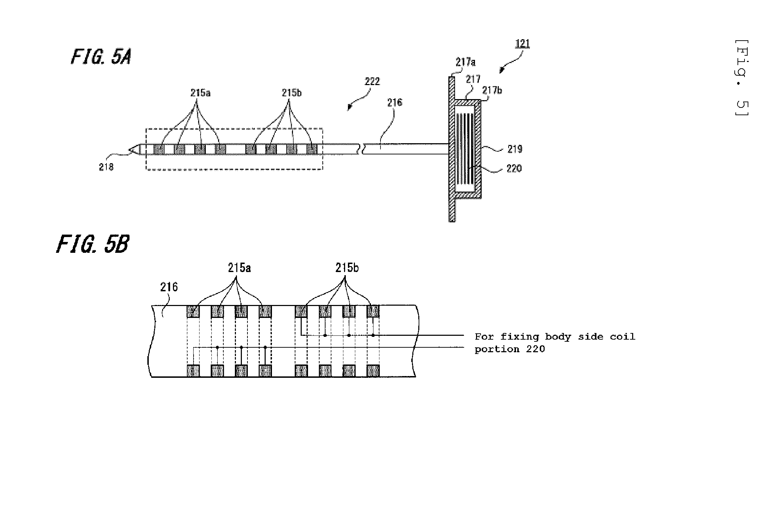

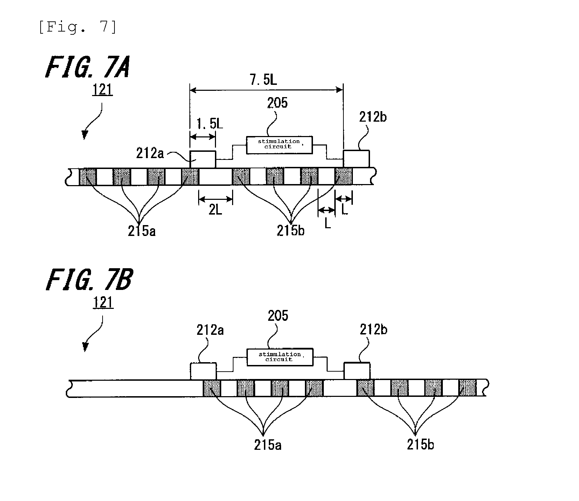

The fixing body 121 is mainly formed of a flexible material such as silicon or polyurethane, and is formed in a bar shape so that it is insertable from the stylet lumen opened to the body 110 of the main body block 122. Furthermore, the internal configuration of the fixing body 121 will be described later by referring to Figs. 5A and 5B to Figs. 7A and 7B.

Next, the internal configuration of the electric stimulation device 101 according to the first embodiment will be described by referring to Figs. 3A and 3B to Figs. 7A and 7B.
Figs. 3A and 3B are enlarged diagram illustrating the main body block according to the first embodiment of the invention.
Fig. 3A is an enlarged external view when the main body block shown in Fig. 1 is seen from the top surface thereof.
Fig. 3B is a cross-sectional view illustrating the cross-section of A-A' of the main body block shown in Fig. 3A.

Further, Figs. 4A to 4G are cross-sectional views illustrating the internal structure at a predetermined position of the main body block according to the first embodiment of the invention in the radial direction.
Fig. 4A is a cross-sectional view illustrating the cross-section of B-B' of the main body block shown in Fig. 3A.
Fig. 4B is a cross-sectional view illustrating the cross-section of C-C' of the main body block shown in Fig. 3A.
Fig. 4C is a cross-sectional view illustrating the cross-section of D-D' of the main body block shown in Fig. 3A.
Fig. 4D is a cross-sectional view illustrating the cross-section of E-E' of the main body block shown in Fig. 3A.
Fig. 4E is a cross-sectional view illustrating the cross-section of F-F' of the main body block shown in Fig. 3A.
Fig. 4F is a cross-sectional view illustrating the cross-section of G-G' of the main body block shown in Fig. 3A.
Fig. 4G is a cross-sectional view illustrating the cross-section of H-H' of the main body block shown in Fig. 3A.

First, the internal configuration of the electrode block 102 will be described.
A pipe 206 is formed of a material having biocompatibility, insulation properties, and flexibility, for example, PTFE (Poly Tetra Fluoro Ethylene) or ETFE (Ethylene-Tetra Fluoro Ethylene copolymer), and is formed in a substantially hollow cylindrical shape. It is desirable that the outer diameter be about 0.1 mm to 1 mm and the inner diameter be substantially equal to or slightly larger than the diameter of the stylet 120 so that the stylet 120 passes inside the pipe 206. One end of the pipe 206 (the end near the front end portion 114) is coupled to a receiving portion 213.

The receiving portion 213 is formed of stainless steel so as to have a substantially cylindrical shape, and a substantially cylindrical hole is formed along the axis in the axial direction. The entire axial direction length and the diameter of the hole are respectively smaller than the entire axial direction length and the outer diameter of the receiving portion 213. Further, it is desirable that the diameter of the hole of the receiving portion 213 be substantially equal to the outer diameter of the pipe 206 so that the pipe 206 is fixed so as to be immovable in the direction perpendicular to the axial direction or the axis. The pipe 206 and the receiving portion 213 are accommodated and fixed into an outer layer portion which includes the bodies 106, the stimulation electrodes 105, and the connector portion 112.

The body 106 is formed of a material having flexibility and biocompatibility, for example, a resin such as silicon or polyurethane. The front end portion 114 of the body 106 is formed in a substantially semi-spherical shape as described above, and it is desirable that the radius be in the range of about 0.5 mm to 1.5 mm. Then, the portion other than the front end portion 114 of the body 106 is formed in a substantially hollow cylindrical shape.

The inner diameter of the portion which is formed in a substantially hollow cylindrical shape is different at the portion in which the body 106 contacts the receiving portion 213 and the portion in which the body 106 contacts the pipe 206. The inner diameter of the portion in which the body 106 contacts the receiving portion 213 is set to be substantially equal to the outer diameter of the receiving portion 213 so as to fix the receiving portion 213. Further, the inner diameter of the portion in which the body 106 contacts the pipe 206 is set to be substantially equal to the outer diameter of the pipe 206 so as to fix the pipe 206. As described above, the four stimulation electrodes 105 are fixed to the substantially hollow cylindrical portions of the body 106 so as to be exposed to the surface of the body 106.

The stimulation electrode 105 is formed of a material having conductivity and biocompatibility, for example, platinum or platinum alloy (alloy of platinum 90%/iridium 10%) so as to have a substantially hollow cylindrical shape. The outer diameter of the stimulation electrode 105 is substantially equal to the outer diameter of the body 106. Further, it is desirable that the inner diameter of the stimulation electrode 105 be a length in which the stimulation electrode 105 and the pipe 206 provided therein does not come into contact with each other. Furthermore, the four stimulation electrodes 105 are defined as the stimulation electrodes 105a, 105b, 105c, and 105d in order from the front end portion 114.

One ends (the ends near the front end portion 114) of conductive wires 202a to 202d adhere to the respective stimulation electrodes 105a to 105d by the solder 203 (see Fig. 4A), and the other ends (the ends near the base end portion 115) of the conductive wires 202a to 202d are electrically connected to the connector portion 112 (see Fig. 2A). Furthermore, the positions other than the adhering positions of the respective conductive wires 202a to 202d using the solder 203 and the electrical connection position to the connector portion 112 are insulated and covered by PTFE or ETFE, and are completely embedded inside the body 106 (see Fig. 4B).

The connector portion 112 is formed of the same material as that of the body 106, and is formed as a notch portion with a step from the outer diameter of the substantially cylindrical body 106. The notch portion is formed by a predetermined distance from the base end portion 115 (see Fig. 2A) in the axial direction. Furthermore, the notch portion is a plane, four connector pins 210 are arranged on the notch portion so as to be exposed therefrom, and the respective conductive wires 202a to 202d are electrically connected to the four connector pins 210.

Next, the internal configuration of the circuit block 103 will be described.
A pipe 207 which is disposed inside the circuit block 103 is the same as the pipe 206 except for the length thereof. The pipe 207 is accommodated and fixed into an outer layer portion which includes the connector portion 107, the body 108 which is continuous to the connector portion 107, and the connector portion 109 which is continuous to the body 108 (see Fig. 2B and Fig. 3B).

The connector portion 107 is formed of the same material (polyurethane or silicon) as that of the body 106 except for an electric connection portion 211 to be described later. In the connector portion 107, a hole substantially having the same shape as that of the outer diameter of the connector portion 112 is opened in the axial direction so as to be connectable to the connector portion 112 of the electrode block 102 (see Fig. 4C). The outer diameter of the connector portion 107 is substantially equal to the outer diameter of the body 106. Further, the connector portion 107 includes the electric connection portion 211 which is independently and electrically connected to each of the four connector pins 210 when the connector portion 107 is connected to the connector portion 112 of the electrode block 102. Furthermore, the connector portion 109 which is provided near the base end portion 117 of the circuit block 103 is the same as the connector portion 107 provided near the front end portion 116, but is not electrically connected to a particularly certain portion.

The body 108 is continuous to the connector portions 107 and 109, and is formed of the same material as those of the connector portions 107 and 109. The body 108 is formed in a substantially hollow cylindrical shape so that the outer diameter thereof is substantially equal to the outer diameter of the connector portion 107 of the circuit block 103 coupled to the connector portion 112 and the inner diameter thereof is substantially equal to the outer diameter of the pipe 207.

The body 108 is embedded with a stimulation circuit 205 which has a small component such as a custom IC mounted on a flexible circuit board and two substantially hollow cylindrical electric power feeding electrodes 212 which are electrically connected to the stimulation circuit 205 (see Figs. 4D and 4E). The stimulation circuit 205 is activated by electric power obtained through the electric power feeding electrodes 212 so as to generate an electric stimulation signal. Furthermore, the electric power feeding electrode 212a and the electric power feeding electrode 212b are electrically connected to a part of the stimulation circuit 205 (a communication unit 302 and a charging unit 308 to be described later). Furthermore, the electric power feeding electrodes 212a and 212b correspond to the first electrical contact points.

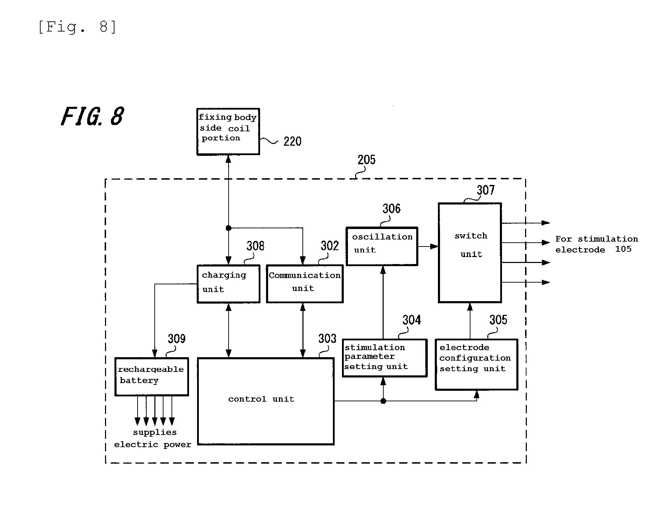

The stimulation circuit 205 is connected to the electric connection portion 211 through conductive wires 204 embedded in the body 108 so that the generated electric stimulation signal is independently supplied to each of the stimulation electrodes 105a to 105d. Furthermore, the electric configuration of the stimulation circuit 205 will be described in Fig. 8.

Next, the internal configuration of the support body 104 will be described.
Pipes 208 and 209 disposed inside the support body 104 are also the same as the pipes 206 and 207 except for the lengths thereof. A valve body 214 is provided between the pipe 208 and the pipe 209 in the axial direction.

The valve body 214 is formed of, for example, an elastic material having biocompatibility (in particular, a soft material is desirable) such as silicon rubber. The valve body 214 includes a first slit which is opened to one end surface near the pipe 208 but is not opened to the other end surface and a second slit which intersects the first slit therein and is opened to one end surface near the pipe 209 but is not opened to the other end surface. By providing the valve body 214, it is possible to prevent a liquid such as a bodily fluid from intruding into the electrode block 102 and the circuit block 103 from the pipe 209 even when the stylet 120 is removed and inserted through the valve body 214.

The pipes 208 and 209 and the valve body 214 are accommodated and fixed into an outer layer portion which includes the connector portion 113, the body 110 which is continuous to the connector portion 113, and the holder portion 111 provided near the base end portion 119 of the body 110.

The connector portion 113 is formed of, for example, polyurethane or silicon, and is formed in the same shape as that of the connector portion 112 of the electrode block 102 (see Fig. 2A). As not in the case of the connector portion 112, since the connector portion 113 does not need to be electrically connected to the outside, there is no need to provide the connector pin 210.

The body 110 is formed of the same material as that of the connector portion 113, and is formed in a substantially hollow cylindrical shape. The outer diameter of the body 110 is substantially equal to the outer diameter of the body 108 of the circuit block 103.

The holder portion 111 is formed of a material such as plastic, and is formed in a substantially hollow cylindrical shape. The inner diameter of the holder portion 111 is substantially equal to the outer diameters of the respective pipes 206 to 209. Further, since the holder portion 111 is a portion which is held at the time of inserting the electric stimulation device 101 into the living body, it is desirable that the outer diameter of the holder portion 111 be twice to three times or more the outer diameter of the body 110.

As described above, the stylet lumen is formed by the receiving portion 213, the pipes 206 to 209, and the valve body 214.

Next, the configuration of the fixing body 121 constituting the electric stimulation device 101 will be described by referring to Figs. 5A and 5B to Figs. 7A and 7B.
Figs. 5A and 5B are diagrams illustrating the configuration of the fixing body.
Fig. 5A is an enlarged diagram illustrating the fixing body, and Fig. 5B is a cross-sectional view illustrating the fixing body of which the portion indicated by the dotted line shown in Fig. 5A is enlarged.

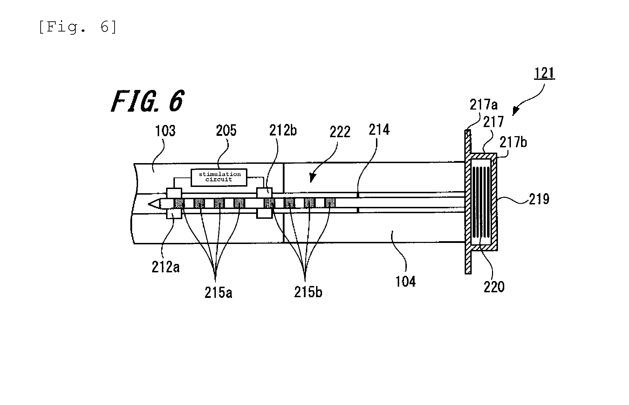

As for the fixing body 121, a front end portion 218 is formed in a substantially conical shape and the other portion is formed in a substantially cylindrical shape. The diameter of the substantially cylindrical portion of the fixing body 121 is formed so as to be slightly smaller than the diameter of the stylet lumen used for the insertion, that is, the inner diameters of the pipes 206 to 209 shown in Fig. 3B. Furthermore, the reason why the front end portion 218 is formed in a substantially conical shape is because the fixing body 121 needs to reach the pipe 208 through the valve body 214 when the front end portion 218 of the fixing body 121 is inserted into the stylet lumen opened to the cross-section of the support body 104, that is, inserted into the pipe 209 (see Fig. 6).

The fixing body 121 includes an insertion portion 222 which is a portion inserted into the stylet lumen and a stopper portion 217 provided in a base end portion 219 of the insertion portion 222.

The insertion portion 222 is formed of a fixing body electrode 215 and a body 216 in which the fixing body electrode 215 is arranged so as to be exposed.

The fixing body electrode 215 is formed of, for example, a conductor such as copper so as to have a substantially hollow cylindrical shape, and includes a fixing body electrode 215a in which four electrodes are arranged at the same interval and a fixing body electrode 215b in which four electrodes are arranged at the same interval. The body 216 is formed of a flexible material such as silicon. In this way, when the fixing body electrode 215 is formed by arranging the plurality of electrodes at the same interval, the body 216 may be flexibly bent, and the insertion portion 222 may be inserted into the stylet lumen of the electric stimulation device 101 implanted to the living body. However, although the number of each of the fixing body electrodes 215a and the fixing body electrodes 215b is set four, this is merely an example, and the number of each of the fixing body electrodes 215a and 215b may be arbitrarily set. Furthermore, the fixing body electrodes 215a and 215b correspond to the second electrical contact points.

Further, the stopper portion 217 is formed of silicon or the like, and as shown in Fig. 5A, includes a disk-like portion 217a to which the base end portion 219 of the body 216 is connected and a substantially hollow cylindrical portion 217b which is formed in the surface near the base end portion 219 of the disk-like portion 217a. A fixing body side coil portion 220 is accommodated in the hollow portion of the substantially hollow cylindrical portion 217b.

The fixing body side coil portion 220 is, for example, a circuit with a coil in which wire is wrapped in a cylindrical shape, and is accommodated so that the axis of the coil and the axis of the stopper portion 217 overlap each other. Then, as shown in Fig. 5B, one end of the coil is connected to the fixing body electrode 215a and the other end of the coil is connected to the fixing body electrode 215b through the conductive wire provided inside the body 216.

Further, since the insertion portion 222 of the fixing body 121 is inserted into the stylet lumen, as shown in Fig. 6, the insertion portion 222 is formed in a substantially cylindrical shape so that the fixing body electrode 215a is connected to the electric power feeding electrode 212a and the fixing body electrode 215b is connected to the electric power feeding electrode 212b. For example, as shown in Fig. 7A, when the electrode lengths and the arrangement of the electric power feeding electrode 212 and the fixing body electrode 215 are set, the electric power feeding electrode 212 and the fixing body electrode 215 are normally connected to each other even when the positional relationship therebetween is different (see Fig. 7B), and both electrodes of the electric power feeding electrode 212a and the electric power feeding electrode 212b are not simultaneously connected to the fixing body electrode 215a or the fixing body electrode 215b so that they are not short-circuited to each other.

### [2. Configuration of stimulation circuit and the like]

Next, in a case where the fixing body electrodes 215a and 215b (see Figs. 6, 7A, and 7B) of the fixing body 121 are respectively connected to the electric power feeding electrodes 212a and 212b (see Fig. 3B) of the circuit block 103, the more specific electric configurations of the stimulation circuit 205 of the circuit block 103 and the fixing body side coil portion 220 of the fixing body 121 will be described by referring to Fig. 8.
Fig. 8 is a block diagram illustrating a function of the stimulation circuit and the fixing body side coil portion according to the example of the first embodiment of the invention.

The stimulation circuit 205 includes the communication unit 302, a stimulation parameter setting unit 304, an electrode configuration setting unit 305, an oscillation unit 306, and a control unit 303. Furthermore, the stimulation circuit includes the charging unit 308, a rechargeable battery 309, and a switch unit 307. Furthermore, the portion of the control unit 303, the charging unit 308, and the rechargeable battery 309 correspond to the power supply unit, and the communication unit 302, the control unit 303, the stimulation parameter setting unit 304, the electrode configuration setting unit 305, the oscillation unit 306, and the switch unit 307 correspond to the electronic circuit.

The rechargeable battery 309 is, for example, a chargeable battery such as a lithium ion battery. Although it is not shown in Fig. 8, the rechargeable battery 309 supplies the stored electric power to the respective blocks constituting the stimulation circuit 205.

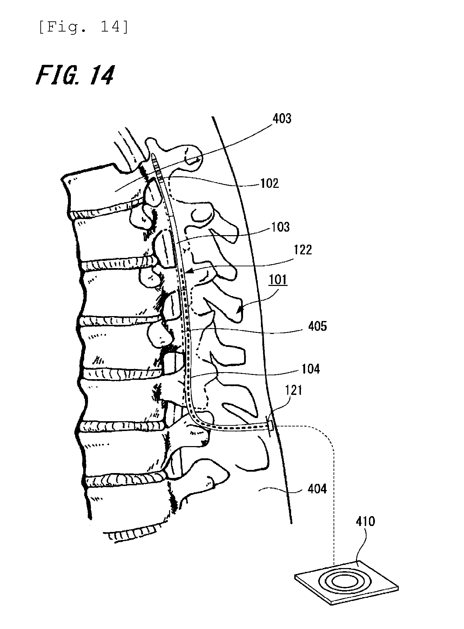

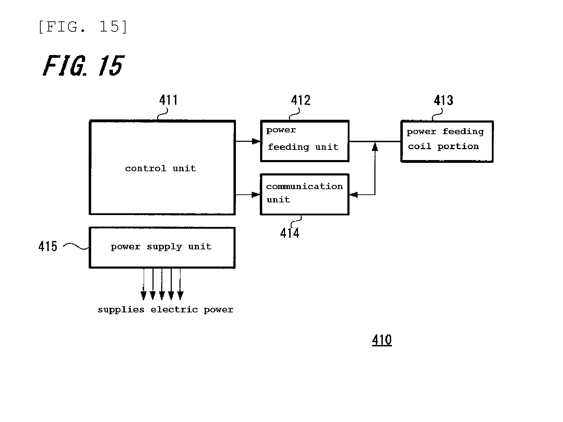

The fixing body side coil portion 220 is, for example, a resonance circuit which includes a coil and a capacitor. The fixing body side coil portion 220 receives a charging electromagnetic wave transmitted from a power feeding patch 410 (to be described later in Fig. 14) at the outside of the living body at the time of charging the rechargeable battery 309. Then, an alternating current which is generated from the fixing body side coil portion 220 with the receiving of the fixing body side coil portion 220 is output to the charging unit 308. Further, the fixing body side coil portion 220 receives an electromagnetic wave with a predetermined information item transmitted from a communication unit 414 (to be described later in Fig. 15) of the power feeding patch 410 to be described later, and outputs the received electromagnetic wave from the fixing body side coil portion 220 to the communication unit 302.

The charging unit 308 includes a rectifying circuit therein, and acquires electric power by converting an alternating current output from the fixing body side coil portion 220 into a direct current. Then, the rechargeable battery 309 is charged by the acquired electric power.

The communication unit 302 demodulates the electromagnetic wave received by the fixing body side coil portion 220, and extracts the information carried by the electromagnetic wave. Then, the extracted information is output to the stimulation parameter setting unit 304 and the electrode configuration setting unit 305 through the control unit 303. The information output to the stimulation parameter setting unit 304 is information on the stimulus strength of the electric stimulation signal (hereinafter, referred to as an "stimulation parameter"), and the information output to the electrode configuration setting unit 305 is information on the electrode configuration (hereinafter, referred to as "electrode configuration information"). Since the stimulus strength of the electric stimulation signal is determined by the pulse voltage, the pulse current, the pulse width, or the frequency of the electric stimulation signal, the stimulation parameter is a signal which represents the value of the pulse voltage or the like. Further, the electrode configuration information is a signal which includes information used to change the polarity of the electric stimulation signal and information used to allow the switch unit 307 to select the stimulation electrode 105 outputting the electric stimulation signal.

The stimulation parameter setting unit 304 generates a stimulus strength change signal used to change the stimulus strength of the electric stimulation signal generated in the oscillation unit 306 based on the stimulation parameter input from the communication unit 302.
The electrode configuration setting unit 305 generates an electrode configuration selection signal used to select the stimulation electrode 105 outputting the electric stimulation signal generated in the oscillation unit 306 based on the electrode configuration information input from the communication unit 302. Furthermore, the stimulus strength change signal output from the stimulation parameter setting unit 304 is output to the oscillation unit 306, and the electrode configuration selection signal output from the electrode configuration setting unit 305 is output to the switch unit 307.

The oscillation unit 306 generates an electric stimulation signal so as to output it to the switch unit 307 based on the stimulus strength change signal input from the stimulation parameter setting unit 304.
The switch unit 307 determines the stimulation electrode 105 which outputs the electric stimulation signal input from the oscillation unit 306 based on the electrode configuration selection signal input from the electrode configuration setting unit 305. Furthermore, the control unit 303 is, for example, a microcomputer or the like, and controls the respective blocks of the stimulation circuit 205.

### [3. Procedure of implanting electric stimulation device]

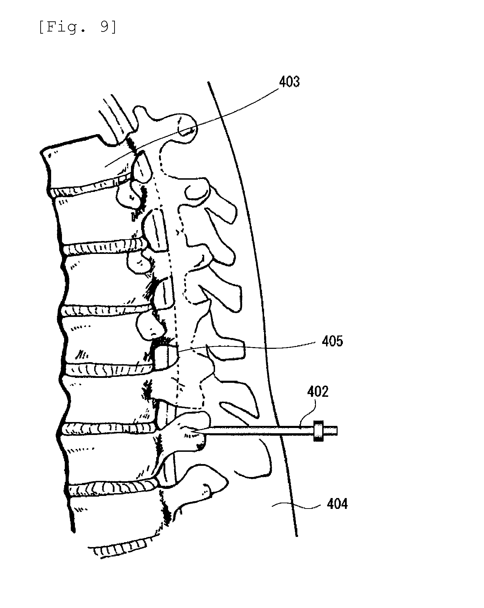

Next, referring to Fig. 9 to Fig. 14, an example of a procedure of implanting the electric stimulation device 101 to, for example, an epidural space and electrically stimulating nerves of the spinal cord by the electric stimulation device 101 will be described.
Fig. 9 to Fig. 14 are longitudinal cross-sectional views illustrating the vicinity of the back of a human body.

First, a doctor determines a target stimulation site of the spinal cord in advance based on the pain pattern of a patient. Then, under X-ray illumination, a puncture is formed from the back side of the patient, and an epidural needle 402 serving as a tubular insertion tool is inserted to an epidural space 405. As the position where the epidural needle 402 is inserted to the epidural space 405, generally, the low-level position of three or more vertebral bodies is selected from the target stimulation site (see Fig. 9).

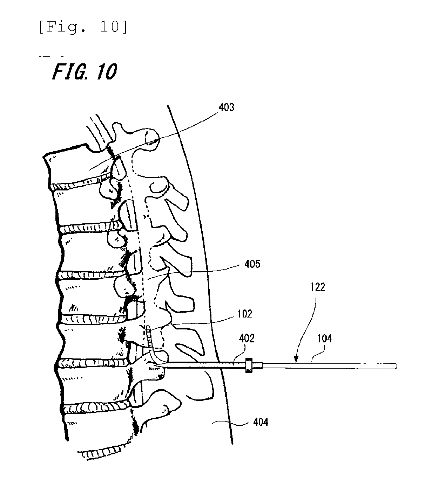

Next, the doctor inserts the stylet 120 into the stylet lumen of the main body block 122 so that the front end of the stylet 120 comes into contact with the receiving portion 213. Then, the main body block 122 is inserted into the living body 404 through the front end portion 114 (see Fig. 1) of the main body block 122 in the epidural needle 402. Then, the main body block 122 is inserted into the epidural space 405 by pressing the base end of the stylet 120 in the axial direction (see Fig. 10, and the holder portion 111 of the main body block 122 and the stylet 120 are not shown).

Subsequently, the doctor directs the main body block 122 upward inside the epidural space 405 by further pressing the base end of the stylet 120 in the axial direction so that the stimulation electrode 105 of the main body block 122 is positioned near the target stimulation site.

Next, the doctor stimulates nerves by operating the power feeding patch 410 to be described later while slightly moving the position of the stimulation electrode 105. At this time, in the stimulation circuit 205 of the main body block 122, a predetermined magnitude of an electric stimulation signal is generated based on the operation on the power feeding patch 410 performed by the doctor, and the generated electric stimulation signal is output to the stimulation electrode 105, thereby stimulating nerves of a portion close to the position of the stimulation electrode 105. Then, the doctor determines the position of the optimal stimulation electrode 105 while asking a reaction with respect to the stimulation on the nerves of the patient.

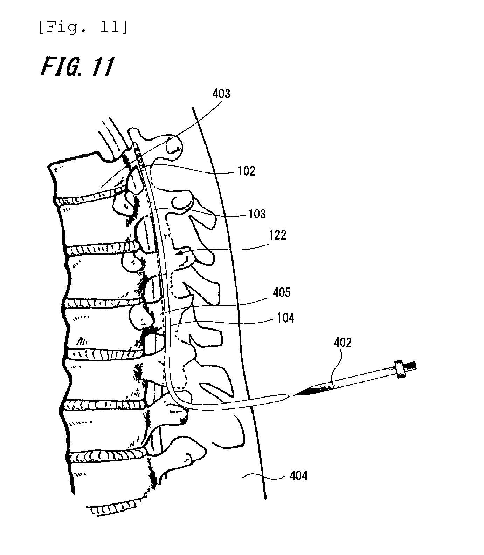

Subsequently, in order that the main body block 122 is completely implanted into the living body 404, the doctor extracts the stylet 120 from the stylet lumen of the main body block 122, cuts the holder portion 111 of the main body block 122, and extracts the epidural needle 402 from the living body 404 (see Fig. 11).

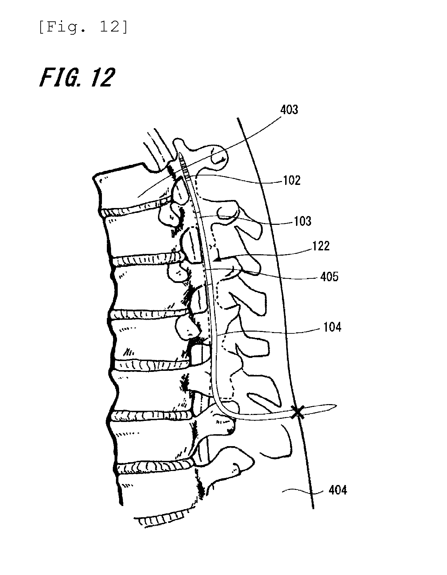

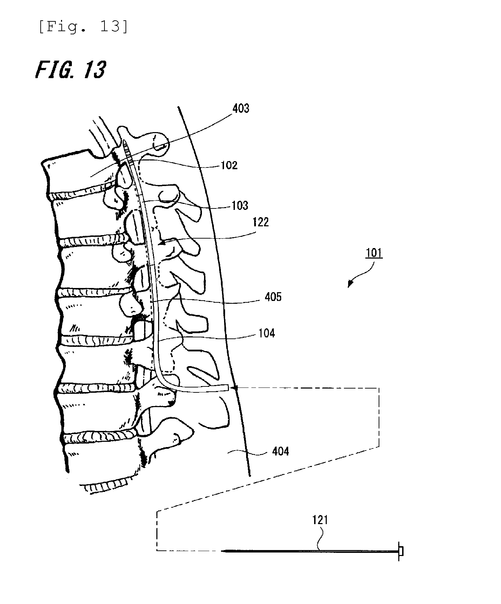

Then, the portion (corresponding to a part of the support body 104) protruding from the living body 404 is cut (see Fig. 12), the cut surface is temporarily fixed to the living body 404 by a thread or the like, and then the front end portion 218 of the fixing body 121 is inserted into the stylet lumen which is opened to the cut surface of the support body 104 (see Fig. 13). Then, the doctor allows the entire body 216 of the fixing body 121 to enter into the stylet lumen by pressing the base end portion 219 of the fixing body 121 in the axial direction of the fixing body 121 (see Fig. 14).

Then, in order that the electric stimulation device 101 is fixed to the living body 404 while being completely implanted thereto, the stopper portion 217 is sewed to the tissue of the living body 404 by a thread (not shown) together with the main body block 122. The treatment is performed so that the electric stimulation device 101 does not move inside the living body 404 or no infection does occur from the insertion opening of the electric stimulation device 101. Further, the treatment is performed so that the base end portion 219 is implanted to the position right below the skin while the coil surface of the fixing body side coil portion 220 of the fixing body 121 of the electric stimulation device 101 is parallel to the skin.

Next, the doctor places the power feeding patch 410 on the body surface so that the coil surface of the power feeding coil portion 413 (to be described later in Fig. 15) of the power feeding patch 410 overlaps the coil surface of the fixing body side coil portion 220. Then, the nerve stimulation is performed by operating the power feeding patch 410. At this time, in the stimulation circuit 205 of the electric stimulation device 101, a predetermined magnitude of an electric stimulation signal is generated based on the operation of the doctor and the generated electric stimulation signal is output to the stimulation electrode 105 so as to stimulate nerves of a portion close to the stimulation electrode 105. Further, the charging to the rechargeable battery 309 (see Fig. 8) of the electric stimulation device 101 is also performed by operating the power feeding patch 410. Furthermore, the power feeding patch 410 will be specifically described later in Fig. 15.

### [4. Configuration of power feeding patch]

Next, the specific electric configuration of the power feeding patch 410 will be described by referring to Fig. 15.
Fig. 15 is a block diagram illustrating a function of the power feeding patch according to the example of the first embodiment of the invention.
The power feeding patch 410 is an example of the power feeding device disposed at the outside of the living body, and includes a control unit 411, a power feeding unit 412, a power feeding coil portion 413, a communication unit 414, and a power supply unit 415.

The control unit 411 includes, for example, a microcomputer, and controls the power feeding unit 412 or the communication unit 414 based on the operation of a user such as a doctor. The operation may be directly performed by an operation unit such as a switch (not shown) disposed in the power feeding patch 410, and may be performed by the communication from the controller (not shown). The power feeding unit 412 generates a charging electromagnetic wave based on the control of the control unit 411 when there is a charging instruction from the user. Then, the generated charging electromagnetic wave is transmitted to the fixing body side coil portion 220 of the electric stimulation device 101 through the power feeding coil portion 413.

The communication unit 414 generates an electromagnetic wave carrying a predetermined information item based on the control of the control unit 411 when there is an instruction for changing the magnitude or the like of the electric stimulation signal from the user. Then, the communication unit 414 transmits the generated electromagnetic wave to the fixing body side coil portion 220 of the electric stimulation device 101 through the power feeding coil portion 413.

Furthermore, the power feeding coil portion 413 may be formed as a coil capable of transmitting an electromagnetic wave to the electric stimulation device 101. For example, a wire may be wrapped in a cylindrical shape.

Further, the power supply unit 415 supplies electric power stored therein to the respective blocks constituting the power feeding patch 410. As the power supply unit 415, a primary battery or a rechargeable battery is used.

### <Description of example of second embodiment of the invention>

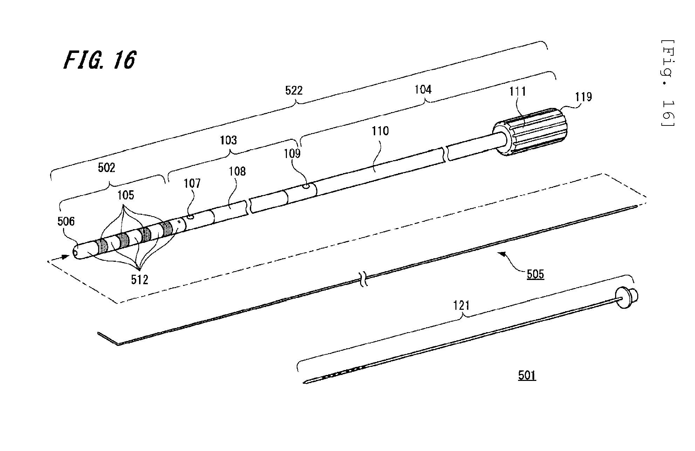

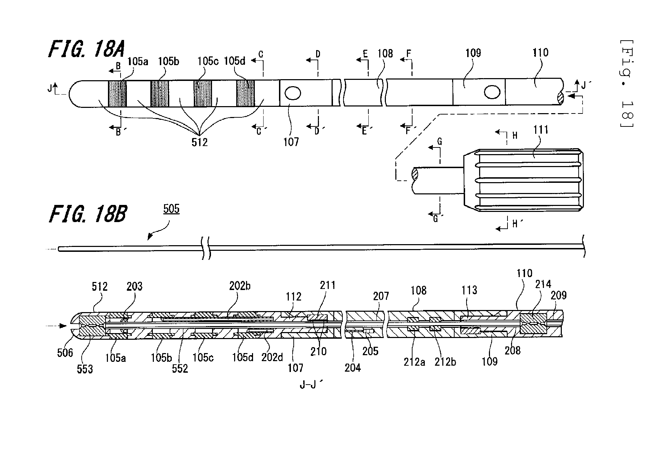

Next, an example of a second embodiment of the invention will be described by referring to Fig. 16 to Figs. 18A and 18B. An electric stimulation device 501 according to the second embodiment shown in Fig. 16 to Figs. 18A and 18B has a configuration in which a cylindrical hole (hereinafter, referred to as a "guide wire lumen") for inserting a guide wire thereinto is formed in the electric stimulation device 101 according to the first embodiment. The same reference numerals will be given to the same parts of the configuration, and the description thereof will be omitted.

### [5. Configuration of electric stimulation device]

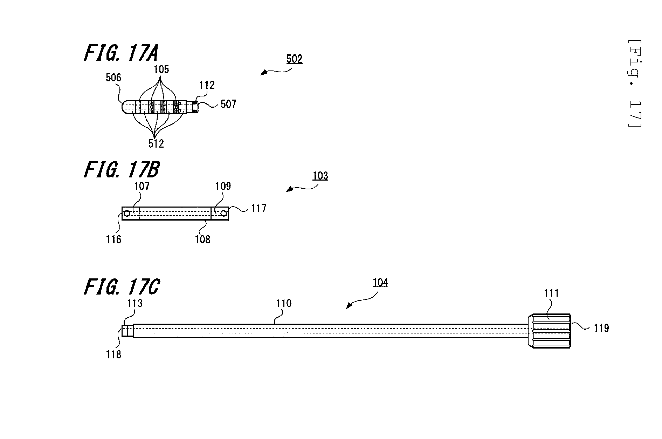

First, a schematic configuration of an electric stimulation device according to the second embodiment will be described by referring to Fig. 16 and Figs. 17A to 17C.
Fig. 16 is a perspective view illustrating the entire electric stimulation device according to the second embodiment.
Figs. 17A to 17C are exploded external views when the main body block constituting the electric stimulation device shown in Fig. 16 is seen from the top surface thereof.

As in the electric stimulation device 101, the electric stimulation device 501 is formed in a substantially elongated cylindrical shape and generates an electric stimulation signal so as to stimulate nerves inside the living body by the stimulation signal. The electric stimulation device 501 has the guide wire lumen along the axis thereof as described above. Then, the guide wire lumen is formed as a penetration hole which is opened to the base end portion 119 and is also opened to a front end portion 506. For this reason, the electric stimulation device 501 has a configuration in which the electrode block 102 of the electric stimulation device 101 of the first embodiment is replaced by a substantially cylindrical electrode block 502 which has a hollow formed in the axial direction. In the second embodiment, the portion obtained by connecting the electrode block 502, the circuit block 103, and the support body 104 to each other is referred to as a main body block 522. Furthermore, the internal configuration of the electrode block 502 will be described in Figs. 18A and 18B.

Next, the internal configuration of the main body block 522 of the electric stimulation device 501 according to the second embodiment will be described by referring to Figs. 18A and 18B and Figs. 4A to 4G.
Figs. 18A and 18B are enlarged diagrams illustrating the main body block and the internal structure thereof in the axial direction according to the second embodiment of the invention.
Fig. 18A is an enlarged external diagram when the main body block shown in Fig. 16 is seen from the top surface thereof.
Fig. 18B is a cross-sectional view illustrating the cross-section of J-J' of the main body block shown in Fig. 18A.

Further, as described above, Figs. 4A to 4G are cross-sectional views illustrating the internal structure at a predetermined position in the radial direction of the main body block 122 according to the first embodiment of the invention, and are also cross-sectional views illustrating the internal structure in the radial direction of the main body block 522 according to the second embodiment.

Here, only the electrode block 502 (see Fig. 17A) constituting the main body block 522 will be described.
A pipe 552 is the same as the pipes 207 to 209 except for the length in the axial direction.
Further, a valve body 553 is the same as the valve body 214. The valve body 553 includes a first slit which is opened to one end surface near the pipe 552 but is not opened to the other end surface and a second slit which intersects the first slit therein and is opened to one end surface near the front end portion 506 of a body 512 but is not opened to the other end surface. Even when a guide wire 505 is removed and inserted through the valve body 553, it is possible to prevent a liquid such as a bodily fluid from intruding into the electrode block 502 and the circuit block 103 from the hole formed in the front end portion 506 of the body 512.

The body 512 is formed of, for example, a material such as silicon or polyurethane, but the front end portion 506 is provided with a substantially cylindrical hole. The diameter of the hole is substantially equal to the outer diameter of the pipe 552. Furthermore, the outer diameter of the body 512 is the same as that of the body 106 (see Figs. 3A and 3B) of the example of the first embodiment. Since the connector portion 112 has the same configuration as that of the first embodiment, the description thereof will be omitted.

### [6. Procedure of implanting electric stimulation device]

Next, a procedure of implanting the electric stimulation device 501 at the time of electrically stimulating nerves of the spinal cord in the electric stimulation device 501 will be simply described. First, the epidural needle 402 is inserted into the epidural space 405 (see Fig. 9), and the guide wire 505 is inserted into the epidural space 405 through the epidural needle 402. Then, the front end of the guide wire 505 is moved to a target stimulation site. Subsequently, the base end of the guide wire 505 is inserted into the front end portion 506 of the main body block 522, and the holder portion 111 is pressed so that the main body block 522 is inserted along the guide wire 505, thereby moving the stimulation electrode 105 of the main body block 522 to the target stimulation site. Then, after an optimal position of the stimulation electrodes 105 is determined by operating the power feeding patch 410, the guide wire 505 is extracted from the main body block 522. Since the other procedures are the same as the procedures described in Fig. 11 to Fig. 14, the description thereof will be omitted.

### <Description of example of third embodiment of the invention>

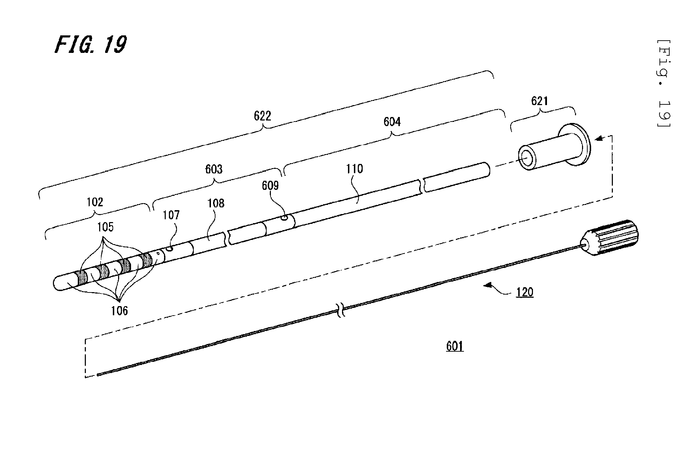

Next, an example of a third embodiment of the invention will be described by referring to Fig. 19 to Fig. 27. Hereinafter, the same reference numerals will be given to the same parts as those of the electric stimulation devices 101 and 501 of the first and second embodiments, and the description thereof will be omitted.

### [7. Configuration of electric stimulation device]

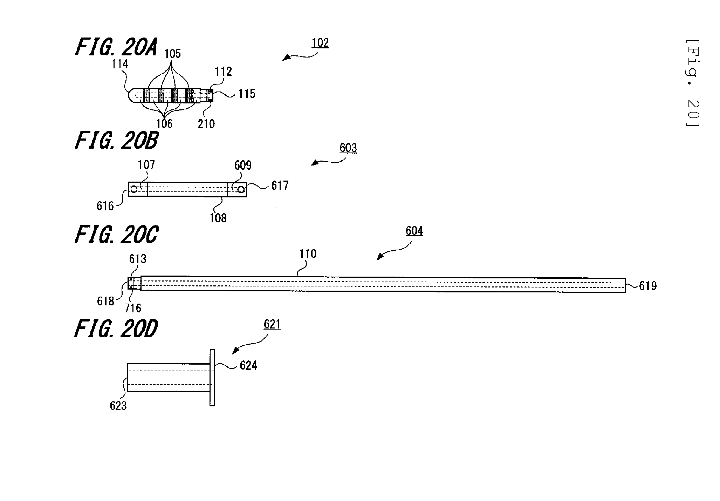

First, a schematic configuration of an electric stimulation device according to the third embodiment will be described by referring to Fig. 19 and Figs. 20A to 20D.

Fig. 19 is a perspective view illustrating the entire electric stimulation device according to the third embodiment.

Figs. 20A to 20D are exploded external views when the electric stimulation device shown in Fig. 19 is seen from the top surface thereof.

As in the respective electric stimulation devices 101 and 501, an electric stimulation device 601 is formed in a substantially elongated cylindrical shape, and generates an electric stimulation signal so as to stimulate nerves and the like inside the living body by the stimulation signal. As shown in Fig. 19 and Figs. 20A to 20D, the electric stimulation device 601 includes a circuit block 603 and a support body 604 instead of the circuit block 103 and the support body 104 of the electric stimulation device 101 (see Fig. 1) according to the first embodiment. Furthermore, a fixing body 621 is provided instead of the fixing body 121. In the third embodiment, the portion obtained by connecting the electrode block 102, the circuit block 603, and the support body 604 to each other is referred to as a main body block 622.

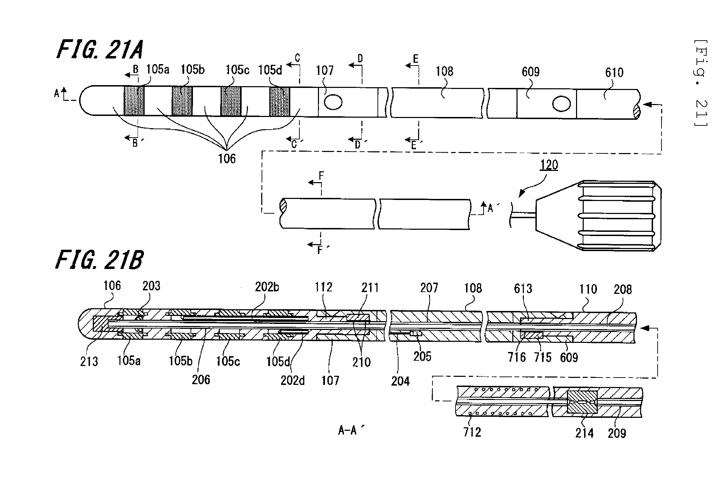

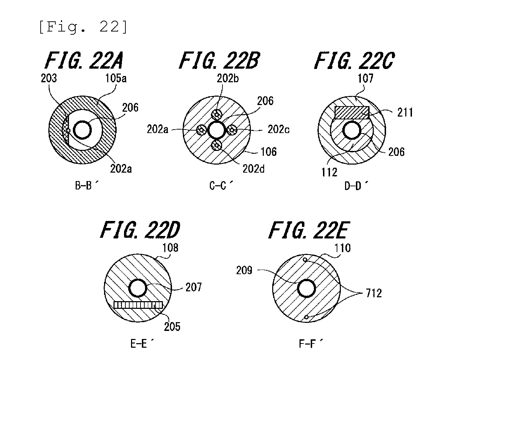

The circuit block 603 is formed in a substantially cylindrical shape so as to have the same diameter as that of the electrode block 102. The circuit block 603 includes the connector portion 107 which is connected to the connector portion 112 of the electrode block 102 so that a front end portion 616 is continuous to the body 106 near the base end portion 115 of the electrode block 102. Further, the circuit block 603 is provided with the body 108 which is continuous to the connector portion 107. Furthermore, a connector portion 609 which connects a base end portion 617 and the support body 604 to each other is formed so as to be continuous to the base end portion 617 of the body 108. Furthermore, the internal configuration of the circuit block 603 will be described later in Figs. 21A, 21B, and 22A to 22E.

The support body 604 includes a connector portion 613 which is connected to the circuit block 603 and the body 110 which is formed in a substantially cylindrical shape so as to have the same diameter as that of the electrode block 102.

The connector portion 613 of the support body 604 is connected to the connector portion 609 of the circuit block 603 so that a front end portion 618 of the body 110 is continuous to the circuit block 603. The body 110 is cuttable so that the electric stimulation device 601 is completely implanted into the living body, and the cut portion, that is, a base end portion 619 of the body 110 is insertable into the fixing body 621. Then, the stylet 120 may be inserted into the stylet lumen which is opened to the base end portion 619. Furthermore, the internal configuration of the support body 604 will be described later in Figs. 21A, 21B, and 22A to 22E.

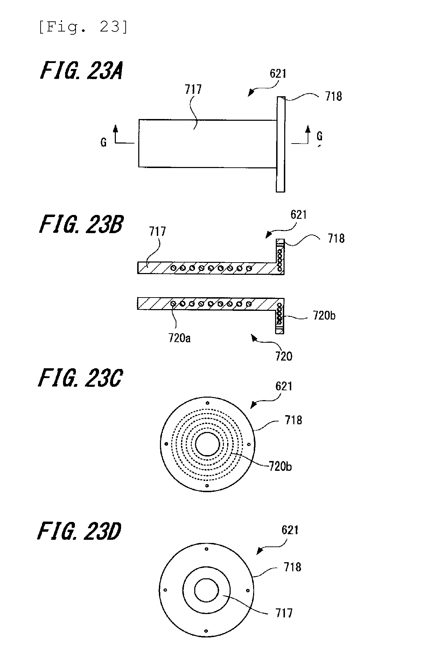

The fixing body 621 is mainly formed of a flexible material such as silicon or polyurethane, and is formed in a substantially cylindrical shape having a hollow for inserting the main body block 622 thereinto. Furthermore, the internal configuration of the fixing body 621 will be described later in Figs. 23A to 23D.

Next, the internal configuration of the main body block 622 according to the third embodiment will be described by referring to Figs. 21A and 21B and Figs. 22A to 22E.
Figs. 21A and 21B are enlarged diagrams illustrating the main body block and the internal structure thereof in the axial direction according to the third embodiment of the invention.
Fig. 21A is an enlarged external view when the main body block shown in Fig. 19 is seen from the top surface thereof.
Fig. 21B is a cross-sectional view illustrating the cross-section of A-A' of the main body block shown in Fig. 21A.

Further, Figs. 22A to 22E are cross-sectional views illustrating the internal structure at a predetermined position in the direction perpendicular to the axis of the main body block according to the third embodiment of the invention.
Fig. 22A is a cross-sectional view illustrating the cross-section of B-B' of the main body block shown in Fig. 21A.
Fig. 22B is a cross-sectional view illustrating the cross-section of C-C' of the main body block shown in Fig. 21A.
Fig. 22C is a cross-sectional view illustrating the cross-section of D-D' of the main body block shown in Fig. 21A.
Fig. 22D is a cross-sectional view illustrating the cross-section E-E' of the main body block shown in Fig. 21A.
Fig. 22E is a cross-sectional view illustrating the cross-section of F-F' of the main body block shown in Fig. 21A.
Here, the circuit block 603 (see Fig. 20B) and the support body 604 (see Fig. 20C) will be only described.

The internal configuration of the circuit block 603 will be described.
The circuit block 603 has a configuration in which the electric power feeding electrodes 212a and 212b (see Fig. 3B) are removed from the circuit block 103 according to the first embodiment and the connector portion 609 is provided instead of the connector portion 109.

The connector portion 609 is the same as the connector portion 107 except for an electric connection portion 715. The electric connection portion 715 is electrically connected to the stimulation circuit 205 so as to supply electric power obtained in a main body side coil portion 712 to be described later to the stimulation circuit 205.

The internal configuration of the support body 604 will be described.
The support body 604 has a configuration in which the main body side coil portion 712 is embedded in the body 110 of the support body 104 according to the first embodiment and the connector portion 613 electrically connected to the main body side coil portion 712 is provided.

The connector portion 613 is formed of, for example, polyurethane or silicon, and is formed in the same shape as that of the connector portion 112 of the electrode block 102 (see Fig. 2A). Then, two connector pins 716 (see Fig. 20C and Fig. 21B) are arranged on the notch portion of the connector portion 613 so as to be exposed wherefrom. The two connector pins 716 are electrically connected to the main body side coil portion 712.

The main body side coil portion 712 which is wrapped about the axis of the body 110 is embedded in the body 110. Each of one end and the other end of the main body side coil portion 712 is electrically and independently connected to each of the two connector pins 716.

Next, the internal configuration of the fixing body 621 will be described by referring to Figs. 23A to 23D.
Figs. 23A to 23D are enlarged diagrams illustrating the fixing body and the internal structure in the axial direction according to the third embodiment of the invention.
Fig. 23A is an enlarged external view when the fixing body shown in Fig. 19 is seen from the top surface thereof.
Fig. 23B is a cross-sectional view illustrating the cross-section of G-G' of the fixing body shown in Fig. 23A.
Fig. 23C is an enlarged diagram of a top surface when the fixing body shown in Fig. 23A is seen from the base end portion.
Fig. 23D is an enlarged diagram of a bottom surface when the fixing body shown in Fig. 23A is seen from the front end portion.

The fixing body 621 includes a stopper portion 718 which is formed of silicon or the like and is continuous to a insertion portion 717 and the insertion portion 717. The fixing body 621 is embedded with a fixing body side coil portion 720. The fixing body side coil portion 720 includes a first coil 720a and a second coil 720b, and one end and the other end of the first coil 720a and one end and the other end of the second coil 720b are respectively connected to each other.

The insertion portion 717 is a portion which is completely implanted into the living body when the electric stimulation device 601 is implanted into the living body, and is formed in a substantially hollow cylindrical shape. It is desirable that the diameter of the hollow portion of the insertion portion 717 be substantially equal to or slightly larger than the diameter of the main body block 622 so that the main body block 622 is insertable into the hollow portion. The insertion portion 717 is embedded with the first coil 720a which is wrapped about the axis of the insertion portion 717.

The stopper portion 718 is formed in a substantially annular shape so as to protrude from one end of the insertion portion 717 to the outside in the radial direction. The inner diameter of the stopper portion 718 is equal to the inner diameter of the insertion portion 717. Further, the outer diameter of the stopper portion 718 is set to be larger than the outer diameter of the insertion portion 717 so that the portion other than the insertion portion 717 of the fixing body 621 does not enter the living body when the electric stimulation device 601 is implanted into the living body. Furthermore, the stopper portion 718 is provided with four holes which are used to fix the electric stimulation device 601 implanted into the living body to the living body through a thread. The stopper portion 718 is embedded with the second coil 720b which is formed in a concentric shape or a spiral shape about the axis of the stopper portion 718. By forming the second coil 720b in a concentric shape or a spiral shape, the thickness of the stopper portion 718 (the length in the axial direction) may be thinned.

### [8. Configuration of stimulation circuit and the like]

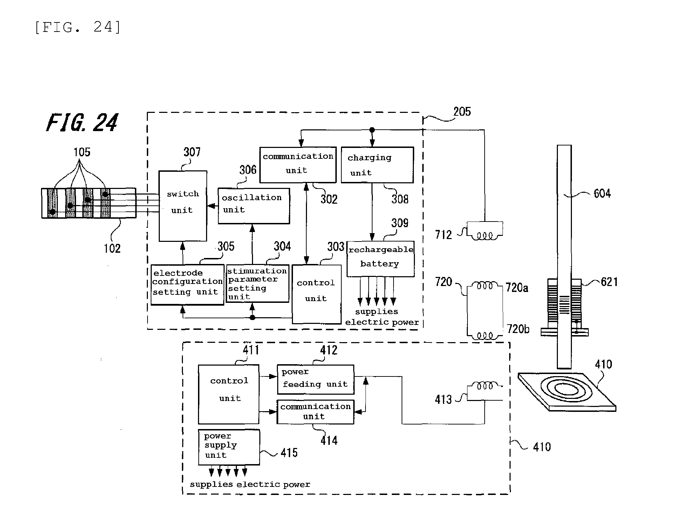

Next, the electric configuration of the electric stimulation device 601 will be described by referring to Fig. 24.
Fig. 24 is a block diagram illustrating a function of the stimulation circuit 205, the main body side coil portion 712, the fixing body side coil portion 720, and the power feeding patch 410 according to the example of the third embodiment of the invention. Furthermore, since the stimulation circuit 205 and the power feeding patch 410 are the same as those of Fig. 8 and Fig. 15, the description thereof will be omitted and herein, only the main body side coil portion 712 and the fixing body side coil portion 720 will be described.

The main body side coil portion 712 is, for example, a resonance circuit which includes a coil and a capacitor. The main body side coil portion 712 receives a charging electromagnetic wave transmitted from the power feeding patch 410 at the outside of the living body through the fixing body side coil portion 720 of the fixing body 621 at the time of charging the rechargeable battery 309. Then, an alternating current which is generated from the main body side coil portion 712 with the receiving in the main body side coil portion 712 is output to the charging unit 308. Further, the main body side coil portion 712 receives an electromagnetic wave carrying a predetermined information item transmitted from the power feeding patch 410 at the outside of the living body through the fixing body side coil portion 720 of the fixing body 621, and outputs the received electromagnetic wave to the communication unit 302. Furthermore, the fixing body side coil portion 720 of the fixing body 621 is a circuit which includes the first and second coils 720a and 720b as described above.

### [9. Procedure of implanting electric stimulation device]

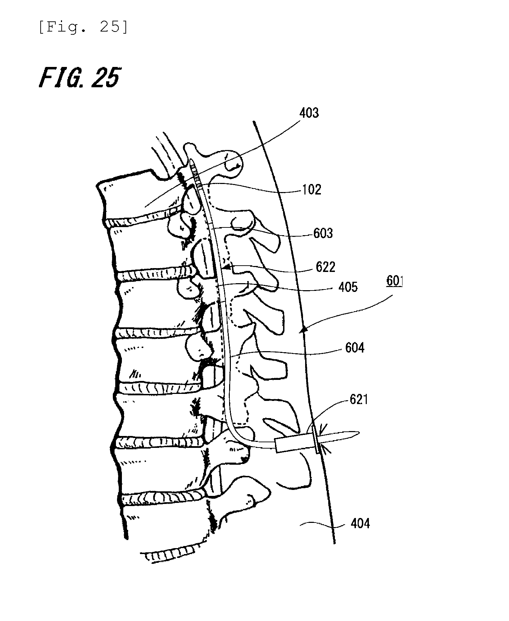

Next, an example of a procedure of implanting the electric stimulation device 601 to, for example, an epidural space and electrically stimulating nerves of the spinal cord using the electric stimulation device 601 will be described. Furthermore, since the procedure until the main body block 622 is implanted into the living body 404 is the same as that of Fig. 9 to Fig. 11, herein, the next procedure will be described by referring to Fig. 25 to Fig. 27.

After the epidural needle 402 is extracted from the living body 404, a doctor inserts the base end portion 619 (see Fig. 20C) of the support body 604 into the front end portion 623 (see Fig. 20D) of the fixing body 621. Then, the doctor presses the fixing body 621 in the axial direction of the main body block 622 so as to completely implant the insertion portion 717 (see Figs. 23A and 23B) into the living body 404, and positions the stopper portion 718 (see Figs. 23A to 23D) onto the hypodermal tissue of a small incision (see Fig. 25). By this treatment, the main body side coil portion 712 of the support body 604 is completely accommodated in the first coil 720a (see Fig. 23B) of the fixing body side coil portion 720 of the fixing body 621. That is, the coil surface of the first coil 720a of the fixing body side coil portion 720 and the coil surface of the main body side coil portion 712 are positioned within the same plane so as to be parallel to each other.

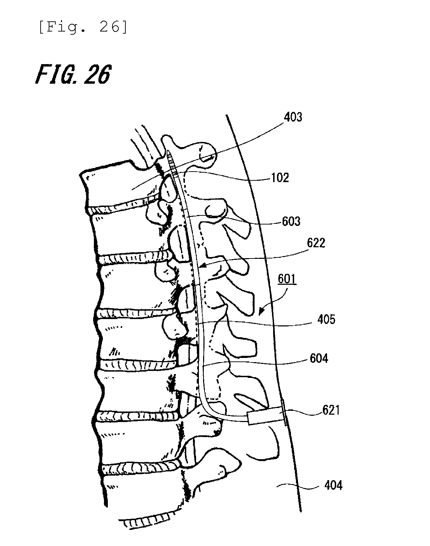

Subsequently, the doctor cuts a portion (corresponding to a part of the support body 604) which protrudes from the stopper portion 718 of the fixing body 621 (see Fig. 26).

After the above-described treatment is completed, the doctor ties the outer periphery of the insertion portion 717 of the fixing body 621 by a thread so as to completely fix the fixing body 621 to the support body 604. Accordingly, it is possible to prevent a bodily fluid or the like from intruding into the stylet lumen which is opened to the support body 604.

Then, the doctor inserts a thread into each of the four holes (see Fig. 23C) formed in the stopper portion 718 of the fixing body 621, and sews the fixing body 621 to a fascia or a connective tissue inside the small incision using the respective threads. The treatment is performed so that the electric stimulation device 601 does not move inside the living body 404. Further, the treatment is performed so that the coil surface of the second coil 720b of the fixing body side coil portion 720 of the fixing body 621 is parallel to the skin. Then, the small incision is sutured so that the electric stimulation device 601 is completely implanted into the living body 404. In this state, the second coil 720b is disposed at a position close to the base end portion 619 of the main body block 622 in relation to the first coil 720a.

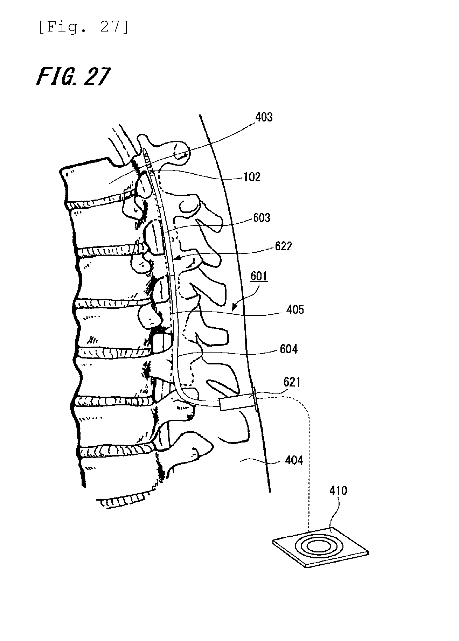

Next, the doctor places the power feeding patch 410 onto the living body surface so that the coil surface of the power feeding patch 410 at the outside of the living body overlaps the coil surface of the second coil 720b of the fixing body side coil portion 720 (see Fig. 27). Then, nerves are stimulated by operating the power feeding patch 410. At this time, in the stimulation circuit 205 of the electric stimulation device 601, a predetermined magnitude of an electric stimulation signal is generated based on the operation of the doctor, the generated electric stimulation signal is output to the stimulation electrode 105, and the nerves of a portion close to the position of the stimulation electrode 105 are stimulated. Further, the charging to the rechargeable battery 309 of the electric stimulation device 601 is also performed by operating the power feeding patch 410.

### <Description of example of fourth embodiment of the invention>

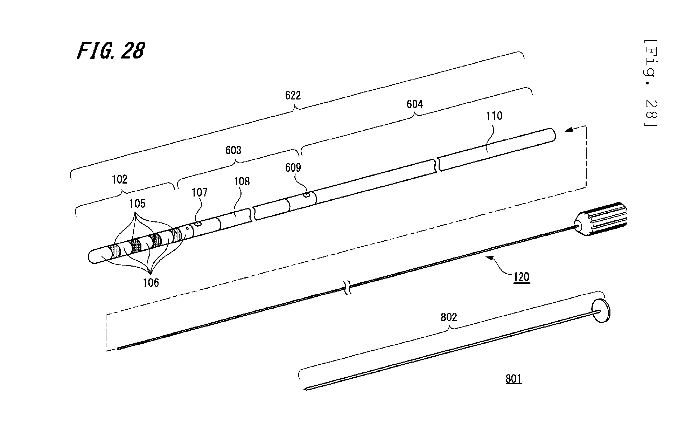

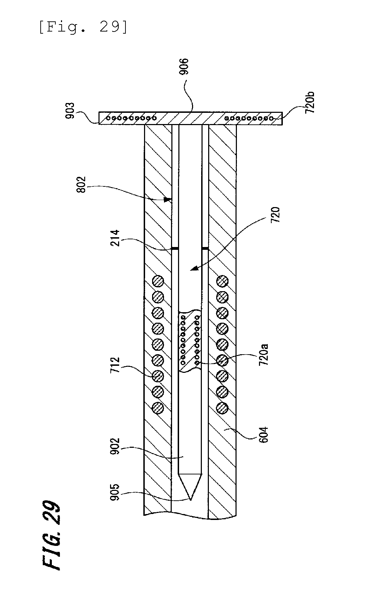

Next, an example of a fourth embodiment of the invention will be described by referring to Figs. 28 and 29. Since an electric stimulation device 801 according to the fourth embodiment shown in Figs. 28 and 29 has substantially the same configuration as that of the electric stimulation device 601 according to the third embodiment, the same reference numerals will be given to the same parts, and the description thereof will be omitted. Further, since the procedure of implanting the electric stimulation device 801 is substantially the same as the procedure of implanting the electric stimulation device 101 according to the first embodiment, the description thereof will be omitted.

### [10. Configuration of electric stimulation device]

First, a schematic configuration of an electric stimulation device according to the fourth embodiment will be described by referring to Fig. 28.
Fig. 28 is a perspective view illustrating the entire electric stimulation device according to the fourth embodiment.
As in the electric stimulation device 601, an electric stimulation device 801 is formed in a substantially elongated cylindrical shape, and generates an electric stimulation signal so that nerves and the like inside the living body are stimulated by the stimulation signal. As shown in Fig. 28, the electric stimulation device 801 has a configuration in which a fixing body 802 is provided instead of the fixing body 621 (see Fig. 19) of the electric stimulation device 601.

The fixing body 802 is mainly formed of a flexible material such as silicon or polyurethane, and is formed in a bar shape so that the fixing body 802 is insertable from the stylet lumen opened to the body 110 of the main body block 622.

Next, the specific configuration of the fixing body 802 will be described by referring to Fig. 29.
Fig. 29 is a cross-sectional view illustrating a configuration in the axial direction of the support body and the fixing body while the fixing body is inserted into the stylet lumen of the support body in the electric stimulation device according to the fourth embodiment.

The fixing body 802 is formed of silicon or the like, and includes an insertion portion 902 which is inserted into the stylet lumen and a stopper portion 903 which is continuous to the insertion portion 902. The fixing body 802 is embedded with the fixing body side coil portion 720. The fixing body side coil portion 720 includes the first coil 720a and the second coil 720b, and one end and the other end of the first coil 720a and one end and the other end of the second coil 720b are respectively connected to each other.

As for the insertion portion 902, a front end portion 905 is formed in a substantially conical shape, and the other portion is formed in a substantially cylindrical shape. The diameter of the substantially cylindrical portion of the insertion portion 902 is formed so as to be slightly smaller than the diameter of the stylet lumen of the main body block 622, that is, the inner diameters of the pipes 206 to 209 shown in Fig. 21B. Furthermore, the reason why the front end portion 905 of the insertion portion 902 is formed in a substantially conical shape is because the insertion portion 902 needs to reach the pipe 208 through the valve body 214 when the front end portion 905 of the fixing body 802 is inserted into the stylet lumen opened to the cross-section of the support body 604, that is, the pipe 209. The insertion portion 902 is embedded with the first coil 720a which is wrapped about the axis of the insertion portion 902.

The stopper portion 903 is formed in a substantially annular shape so as to protrude from one end of the insertion portion 902 to the outside in the radial direction. The diameter of the stopper portion 903 is set to be larger than the diameter of the insertion portion 902 so that the portion other than the insertion portion 902 of the fixing body 802 does not enter into the stylet lumen when the fixing body 802 is inserted into the stylet lumen of the main body block 622. Although the stopper portion 903 is embedded with the second coil 720b which is formed in a concentric shape or a spiral shape about the axis of the stopper portion 903, the thickness of the stopper portion 903 (the length in the axial direction) may be thinned by forming the second coil 720b in a concentric shape or a spiral shape. Furthermore, as in the stopper portion 718 (see Figs. 23A to 23D), it is needless to mention that the stopper portion 903 is provided with four holes which are used to fix the electric stimulation device 801 implanted into the living body to the living body by a thread.

Since the insertion portion 902 of the fixing body 802 is inserted into the stylet lumen of the main body block 622, as shown in Fig. 29, the first coil 720a of the fixing body side coil portion 720 is completely accommodated in the main body side coil portion 712. That is, the coil surface of the first coil 720a of the fixing body side coil portion 720 and the coil surface of the main body side coil portion 712 are positioned within the same plane so as to be parallel to each other. For this reason, in the state shown in Fig. 29, when the power feeding patch 410 is placed on the body surface so that the coil surface of the power feeding patch 410 at the outside of the living body overlaps the coil surface of the second coil 720b of the fixing body side coil portion 720, the electromagnetic wave generated from the power feeding patch 410 is received by the main body side coil portion 712 through the fixing body side coil portion 720. Then, in accordance with the electromagnetic wave from the power feeding patch 410, the electric stimulation signal is generated by the oscillation unit 306 or the rechargeable battery 309 is charged by the charging unit 308.

As described above, in the above-described respective embodiments, it is possible to dispose the fixing body side coil portion in the base end portion of the cut support body. For this reason, when the electric stimulation device is completely implanted into the living body, the fixing body side coil portion may be implanted to the vicinity right below the skin, and the efficient power feeding and communication may be performed between the power feeding coil portion of the power feeding patch at the outside of the living body and the fixing body side coil portion. That is, it is possible to reduce the magnitude of the electromagnetic wave which is necessary for the fixing body side coil portion to generate electric power through electromagnetic induction. As a result, there is an effect that the power feeding patch transmitting the electromagnetic wave to the fixing body side coil portion may be decreased in size.

Further, in the above-described respective embodiments, since the fixing body is connected to the cut support body, the coil surface of the fixing body side coil portion of the fixing body becomes substantially perpendicular to the axial direction of the support body. Accordingly, when the electric stimulation device is completely implanted into the body, the coil surface of the fixing body side coil portion of the fixing body may be easily made to be parallel to the skin. As a result, it is possible to easily align the axis of the power feeding patch for charging the electric stimulation device disposed at the outside of the living body or setting parameters, and more easily perform the efficient power feeding and communication. Thus, it is possible to further reduce the magnitude of the electromagnetic wave necessary for the fixing body side coil portion to generated electric power through electromagnetic induction.

Further, in the above-described respective embodiments, since the main body block is provided with the stylet lumen or the guide wire lumen, the stylet or the guide wire may be used at the time of implanting the electric stimulation device into the living body. For this reason, it is possible to easily implant the electric stimulation device into the living body, and further improve the accuracy of the arrangement of the stimulation electrode into the living body.

Further, in the above-described respective embodiments, the support body may be cut at a desired position. The length of the support body, that is, the length in the axial direction of the main body block may be changed to a certain extent. Accordingly, the electric stimulation device may be completely implanted into the living body. As a result, the infection or the like from the insertion opening of the electric stimulation device may be prevented.

Further, in the above-described first and second embodiments, even when the support body is cut at a certain position, if the fixing body is inserted into the stylet lumen/guide wire lumen opened to the cut surface, the fixing body electrode of the fixing body and the electric power feeding electrode of the circuit block may be connected to each other. That is, electric power is supplied to the fixing body side coil portion and the charging unit of the stimulation circuit. For this reason, even when the support body is cut at a certain position, the electric power generated in the fixing body side coil portion may be supplied to the respective blocks of the stimulation circuit.

Further, in the above-described third and fourth embodiments, when the fixing body is fixed to the body of the support body, the coil surface of the first coil of the fixing body side coil portion provided in the fixing body and the coil surface of the main body side coil portion are positioned within the same plane so as to be parallel to each other. Accordingly, the efficient power feeding and communication may be performed between the main body side coil portion and the fixing body side coil portion.

### <Description of modified example>

Furthermore, in the above-described respective embodiments, the main body block is configured to directly pass through the epidural needle at the time of inserting the electric stimulation device into the living body. However, when a flexible cannula is guided to the vicinity of the stimulus portion through the epidural needle in advance and then the main body block of the electric stimulation device is inserted into the living body through the cannula, it is possible to further improve the accuracy of the arrangement of the stimulation electrode into the living body.

Further, in the above-described respective embodiments, the electrode block, the circuit block, and the support body are respectively connected to each other by the connector so as to be attachable to and detachable from each other. However, instead of the connector, the electrode block and the circuit block may be integrated with each other in advance, the circuit block and the support body may be integrated with each other in advance, or all components may be integrated with each other in advance.

Further, in the above-described respective embodiments, the rechargeable battery (the secondary battery) is used as the power supply of the stimulation circuit, but a capacitor may be used instead of the rechargeable battery so as to operate the stimulation circuit while receiving the electric power from the power feeding patch at the outside of the living body at all times.

Further, in the above-described first, third, and fourth embodiments, the support body, the circuit block, and the electrode block are provided with the stylet lumen which is formed in the axial direction. However, the stylet lumen may be formed only in the support body and the circuit block or may be formed only in the support body. Further, in the third and fourth embodiments, the lumen (the guide wire lumen) may be formed from the base end portion of the support body to the front end portion of the electrode block. By providing the guide wire lumen, the main body block may be implanted into the body using the guide wire. Furthermore, it is needless to mention that the lumen may not be provided.

Further, in the above-described respective embodiments, the main body block is formed in a substantially elongated cylindrical shape, but the invention is not limited to the substantially cylindrical shape. Any shape may be used if the main body block is formed in an elongated shape.

Further, in the above-described first and second embodiments, as shown in Fig. 6, the fixing body electrodes 215a and 215b are formed by arranging the plurality of electrodes at the same interval. However, instead of this configuration, each of the electric power feeding electrodes 212a and 212b may be formed by a plurality of electrodes.

Further, in the above-described first and second embodiments, the stopper portion 217 may be electrically and mechanically connected to the base end portion 219 of the body 216 of the fixing body 121 through a connector. By preparing various lengths of insertion portions 222, as shown in Fig. 6, the fixing body electrodes 215a and 215b may be reliably and respectively connected to the electric power feeding electrodes 212a and 212b.

Further, in the above-described first and second embodiments, the electric power feeding electrode 212 may be provided in the support body instead of forming the electric power feeding electrode 212 in the circuit block. In this case, the electric power feeding electrode 212 needs to be electrically connected to the stimulation circuit 205 through the connector portions 113 and 109.

Further, in the above-described third and fourth embodiments, the main body side coil portion is provided in the support body, but may be provided in the circuit block.

Further, in the above-described third and fourth embodiments, the second coil which is embedded in the stopper portion of the fixing body is formed in a concentric shape or a spiral shape about the axis of the fixing body, but may be wrapped about the axis of the stopper portion of the fixing body.

Further, the stylet lumen may be provided in the axial direction of the fixing body so that the fixing body (see Fig. 5A and Fig. 29) according to the above-described first, second, and fourth embodiments is inserted into the stylet lumen or the guide wire lumen using the stylet. In this case, it is desirable that the lumen be opened to the base end portion of the fixing body.

Further, in the above-described fourth embodiment, the base end portion 906 of the insertion portion 902 of the fixing body 802 may be electrically and mechanically connected to the stopper portion 903 through a connector. By preparing various lengths of insertion portions 902, as shown in Fig. 29, the first coil 720a of the fixing body side coil portion 720 may be reliably accommodated inside the main body side coil portion 712.

Incidentally, in the above-described respective embodiments, the portion obtained by connecting the electrode block and the circuit block to each other corresponds to the stimulation circuit block of claims.

While the examples of the respective embodiments of the invention have been described, the invention is not limited to the examples of the above-described respective embodiments, and it is needless to mention that other modification examples and application examples are included without departing from the spirit of the invention according to claims.

### Reference Signs List

- 101, 501, 601, 801:: electric stimulation device
- 102, 502:: electrode block
- 103, 603:: circuit block
- 104, 604:: support body
- 105:: stimulation electrode
- 106, 108, 110, 216, 512:: body
- 107, 109, 112, 113, 609, 613:: connector portion
- 111:: holder portion
- 120:: stylet
- 121, 621, 802:: fixing body
- 122, 522, 622:: main body block
- 205:: stimulation circuit
- 206, 207, 208, 209, 552:: pipe
- 210, 716:: connector pin
- 211, 715:: electric connection portion
- 212:: electric power feeding electrode
- 213:: receiving portion
- 214, 553:: valve body
- 215:: fixing body electrode
- 217, 718, 903:: stopper portion
- 220, 720:: fixing body side coil portion
- 222, 717, 902:: insertion portion
- 302:: communication unit
- 303:: control unit
- 304:: stimulation parameter setting unit
- 305:: electrode configuration setting unit
- 306:: oscillation unit
- 307:: switch unit
- 308:: charging unit
- 309:: rechargeable battery
- 402:: epidural needle
- 404:: living body
- 405:: epidural space
- 410:: power feeding patch
- 411:: control unit
- 412:: power feeding unit
- 413:: power feeding coil portion
- 414:: communication unit
- 415:: power supply unit
- 505:: guide wire
- 712:: main body side coil portion
- 720a:: first coil
- 720b:: second coil

## Claims

1. An electric stimulation device that is formed in a bendable elongated shape and is implanted into a living body, the electric stimulation device comprising:
a main body block that includes a stimulation electrode stimulating nerves or muscles inside the living body, a stimulation circuit block with a stimulation circuit electrically connected to the stimulation electrode and applying a stimulation signal to the stimulation electrode, and a support body connected to the stimulation circuit block and holding the implant position of the stimulation electrode inside the living body; and
a fixing body that is formed so as to be connectable to the base end portion of the main body block,
wherein the fixing body includes a fixing body side coil portion that receives an electromagnetic wave transmitted from an external device, and
wherein when the fixing body is connected to the main body block, the fixing body side coil portion is electrically connected to the stimulation circuit, so that power feeding and/or communication with respect to the stimulation circuit in response to the electromagnetic wave are enabled.

2. The electric stimulation device according to claim 1,
wherein the fixing body side coil portion is formed so that a coil surface is disposed in parallel to a body surface while the electric stimulation device is implanted into the living body.

3. The electric stimulation device according to claim 1 or 2,
wherein the support body is cuttable, and even when the support body is cut by a predetermined length, the stimulation circuit and the fixing body side coil portion are electrically connected to each other by connecting the fixing body to the main body block including the cut support body.

4. The electric stimulation device according to any one of claims 1 to 3,
wherein at least the support body of the main body block is provided with a lumen that is opened to the base end portion of the support body,
wherein the inside of the lumen is provided with at least two first electrical contact points electrically connected to the stimulation circuit,
wherein the fixing body includes a body having at least two second electrical contact points electrically connected to the fixing body side coil portion and insertable into the lumen, and
wherein when the body is inserted into the lumen, the first and second electrical contact points are connected to each other, so that the stimulation circuit and the fixing body side coil portion are electrically connected to each other.

5. The electric stimulation device according to claim 4, wherein each of the first electrical contact points includes a plurality of electrodes

6. The electric stimulation device according to claim 4, wherein each of the second electrical contact points includes a plurality of electrodes.

7. The electric stimulation device according to claim 5 or 6,
wherein the plurality of electrodes have the same length and are arranged in the body at the same interval.

8. The electric stimulation device according to any one of claims 4 to 7,
wherein the main body block includes a bodily fluid intrusion preventing structure that is provided near the base end portion in relation to the installation positions of the first electrical contact points in the lumen.

9. The electric stimulation device according to any one of claims 1 to 3,
wherein the main body block includes a main body side coil portion that is connected to the stimulation circuit,
wherein the fixing body side coil portion includes a first coil and a second coil electrically connected to the first coil and receiving the electromagnetic wave, and
wherein when the fixing body is connected to the main body block, the coil surface of the main body side coil portion and the coil surface of the first coil are parallel to each other, and the second coil is disposed at a position close to the base end portion of the main body block in relation to the first coil.

10. The electric stimulation device according to claim 9, wherein the first coil is disposed inside the main body side coil portion or the main body side coil portion is disposed inside the first coil.

11. The electric stimulation device according to claim 9 or 10,
wherein the fixing body is formed in a cylindrical shape having a hollow into which the support body is insertable,
wherein the first coil is wrapped about the axis of the fixing body,
wherein when the support body is inserted into the fixing body so that the main body block and the fixing body are connected to each other, the stimulation circuit and the second coil are electrically connected to each other through the first coil and the main body side coil portion.

12. The electric stimulation device according to claim 9 or 10,
wherein the lumen opened to the base end portion of the support body is formed up to the vicinity of at least the main body side coil portion,
wherein the fixing body is formed in a bar shape so as to be insertable into the lumen,
wherein the first coil is wrapped about the axis of the fixing body, and
wherein when the fixing body is inserted into the lumen so that the main body block and the fixing body are connected to each other, the stimulation circuit and the second coil are electrically connected to each other through the first coil and the main body side coil portion.

13. The electric stimulation device according to any one of claims 9 to 12,
wherein the second coil is disposed in the base end portion of the fixing body.

14. The electric stimulation device according to any one of claims 1 to 13,
wherein the fixing body side coil portion is formed in a concentric shape or a spiral shape about the axis of the fixing body.

15. The electric simulation device according to any one of claims 1 to 14,
wherein the stimulation circuit includes a chargeable secondary battery.

16. The electric stimulation device according to any one of claims 1 to 14,
wherein the stimulation circuit includes a storage capacitor.
